# FASCICULE DE BREVET EUROPEEN

(11) **EP 3 302 576 B1**
(45) Date de publication et mention de la délivrance du brevet: **28.04.2021**
(21) Numéro de dépôt: 16725119.8
(22) Date de dépôt: 24.05.2016
(51) Int. Cl.: A61K 49/00, C07K 7/64

(54) **TRACEUR FLUORESCENT CIBLANT MULTIVALENT DANS LE PROCHE INFRAROUGE POUR IMAGERIE OPTIQUE.**
MULTIVALENTER TARGETING-FLUORESZENZTRACER IM NAHINFRAROTBEREICH FÜR OPTISCHE BILDGEBUNG
MULTIVALENT TARGETING FLUORESCENT TRACER IN THE NEAR INFRARED RANGE FOR OPTICAL IMAGING

(30) Priorité: 25.05.2015 FR 1554668
(43) Date de publication de la demande: 11.04.2018
(73) Titulaire: Fluoptics, 38040 Grenoble (FR)
(72) Inventeur: GAYET, Pascal, 38040 Grenoble (FR)
(74) Mandataire: Marks & Clerk France
(86) Numéro de dépôt international: PCT/EP2016/061711
(87) Numéro de publication internationale: WO 2016/189004

(56) Documents cités:
- US-A1- 2014 271 482
- Elisabeth Garanger: "Conception, Synthèse et Caractérisation de Nouveaux Systèmes de Guidage et de Vectorisation pour la Cancérologie", Thèse - Université Joseph Fourier - Grenoble I, 26 juillet 2005 (2005-07-26), XP055262866, Extrait de l'Internet: URL:https://tel.archives-ouvertes.fr/tel-0 0009834/document [extrait le 2016-04-05]

## Description

La présente invention concerne des composés tels que des sondes de fluorescence dans le domaine du proche infrarouge. Ces composés comprennent des molécules de ciblage de tissus ou organes cibles, un fluorophore et un support sur lequel sont fixés les molécules de ciblage et le fluorophore.

### ARRIERE PLAN TECHNOLOGIQUE

L'imagerie de fluorescence est en plein essor dans de nombreuses applications chirurgicales. Plusieurs dispositifs médicaux sont sur le marché permettant la détection et la visualisation d'un des seuls fluorophores disposant d'une autorisation de mise sur le marché : le vert d'indocyanine. Toutefois, ce fluorophore est une molécule non spécifique de tissus ou d'organes cibles ce qui restreint son champ d'application.

L'imagerie médicale est une technique prometteuse dans les interventions chirurgicales. Elle peut, par exemple, servir à guider le chirurgien lors de son intervention. Cette technique repose sur l'administration au patient d'un traceur fluorescent. En l'espèce, il s'agit d'un traceur fluorescent ciblant des tissus ou organes nécessitant l'intervention chirurgicale.

Le principe repose sur un éclairement par une source lumineuse d'un traceur fluorescent, préalablement administré au patient, spécifique de tissus ou organes cibles. L'éclairement a pour effet d'exciter ledit traceur fluorescent qui émet alors à son tour un rayonnement à une longueur d'onde donnée. Les principales applications sont dans le domaine du proche infrarouge entre 700 nm et 1000 nm. En effet, cette fenêtre optique correspond au domaine de longueurs d'ondes dans lequel les tissus biologiques absorbent le moins.

L'intérêt des traceurs fluorescents est qu'ils permettent d'associer des propriétés d'au moins deux classes de molécules telles que des propriétés de fluorescence d'une part, et, des propriétés de ciblage de tissus ou organes spécifiques d'autre part.

Les demandes de brevet américaines US20140271482 de Low et US 20140275533 de Kularatne notamment présentent des molécules fluorescentes monovalentes ciblant des tissus spécifiques. Une molécule est dite monovalente lorsqu'elle ne fixe qu'un exemplaire de molécule de ciblage. En l'espèce, les sondes décrites ciblent des zones liées à des maladies inflammatoires. Ces demandes ne divulguent pas de support multivalent permettant la fixation de plusieurs molécules de ciblage, par exemple.

L'objet de la présente invention est de proposer une molécule ou traceur ciblant des tissus ou organes spécifiques. Plus précisément, l'objet de la présente invention est de proposer un traceur fluorescent comprenant un support multivalent fluorescent présentant des points d'ancrage pour des molécules de ciblage.

Dans cette optique, le brevet EP1539804 de P. Dumy décrit un procédé de synthèse d'un support peptidique ou châssis moléculaire.

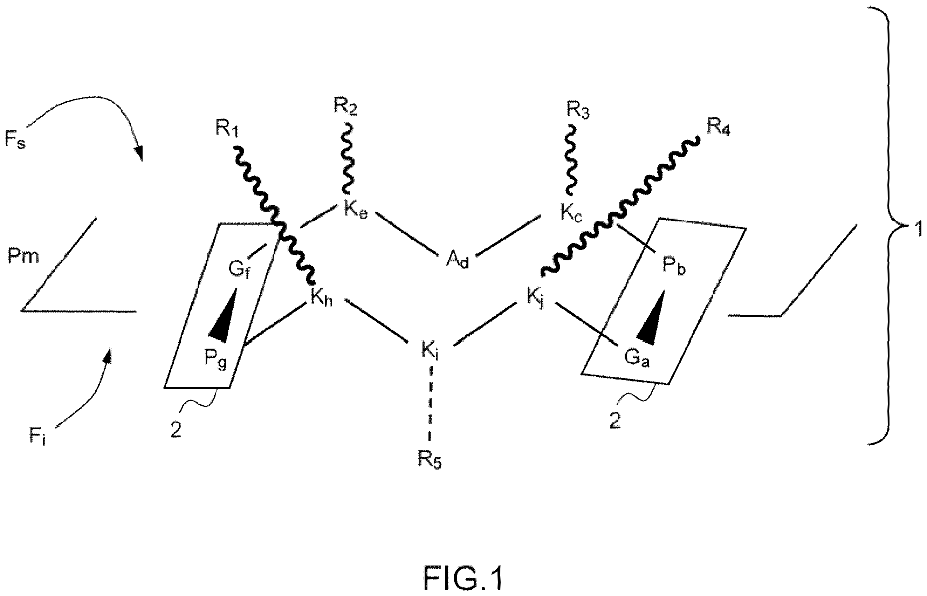

La figure 1 représente un exemple de châssis moléculaire. Il s'agit d'un châssis moléculaire **1,** plus connu sous le nom de châssis RAFT, acronyme de « Regioselectively Adressable Functionnalized Templates », en langue anglaise ou châssis fonctionnalisable régio-sélectivement.

Il s'agit d'un décapeptide cyclique comprenant la séquence de résidus d'acides aminés : [-Glycineₐ-Proline_{b}-Lysine_{c}-Alanine_{d}-Lysineₑ-Glycine_{f}-Proline_{g}-Lysineₕ-Lysineᵢ-Lysineⱼ-], les enchaînements des acides aminés [-Glycine _{a ; f} - Proline _{b ; g}-] formant des coudes **2** de sorte que la configuration du châssis moléculaire **1** présente un plan moyen **Pm** définissant une face dite supérieure **Fₛ** comprenant quatre résidus Lysine **K_{c}, Kₑ, Kₕ** et **Kⱼ** du décapeptide cyclique et une face dite inférieure **Fᵢ,** opposée à la face supérieure **Fₛ** comprenant au moins une Lysine Kᵢ.

On entend par plan moyen **Pm** le plan pour lequel la somme des distances entre le plan moyen **Pm** et les résidus d'acides aminés est minimale.

La préparation du châssis moléculaire **1** comprend une première étape de synthèse du décapeptide linéaire, la méthode d'élongation mise en œuvre afin d'obtenir le décapeptide linéaire à partir d'une glycine ancrée dans une résine étant décrite dans l'état de la technique « P. Dumy et al. Tetrahedron Lett. 1995, 36, 1255-1258 *»,* une deuxième étape de cyclisation intramoléculaire du décapeptide linéaire de sorte à obtenir un châssis moléculaire cyclique et une troisième étape de fonctionnalisation du châssis moléculaire cyclique **1** selon les fonctionnalités souhaitées.

Le décapeptide cyclique RAFT ainsi réalisé est multivalent, il peut être associé de manière contrôlée à deux domaines fonctionnels indépendants : un domaine dédié au ciblage de zones d'intérêt, telles que des zones exprimant des intégrines, et un domaine pour la détection.

On entend par « intégrines » des hétéro-dimères transmembranaires des récepteurs de surface cellulaire qui jouent un rôle dans les processus d'adhésion cellule-cellule et cellule-matrice extracellulaire. Les intégrines jouent un rôle important dans le rattachement des cellules à leur environnement, et, en particulier, au réseau de protéines de la matrice extracellulaire. L'intégrine αᵥβ₃ est une intégrine identifiée comme spécifique des zones d'angiogénèse. Il a été démontré que cette intégrine était surexprimée par les cellules endothéliales des vaisseaux néo-angiogéniques, elle a également été mise en évidence sur de nombreuses lignées de cellules tumorales humaines.

De nombreuses intégrines interagissent avec leurs substrats protéiques via la séquence de résidus d'acides aminés -RGD- acronyme de « Arginine-Glycine-Acide Aspartique ». Cette séquence RGD est un motif commun présent sur la plupart des protéines de la matrice extracellulaire.

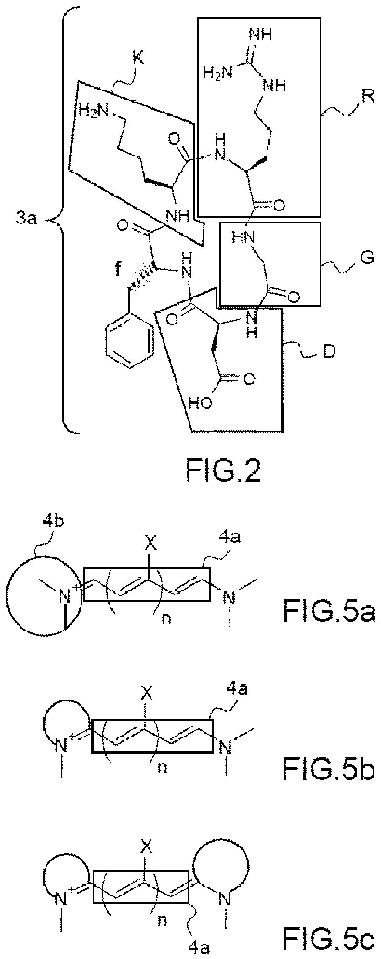

La figure 2 présente la formule chimique d'une molécule de ciblage **3** comprenant un pentapeptide cyclique spécifique de l'intégrine αᵥβ₃ comprenant la séquence de résidus d'acides aminés **R, G** et **D,** acronyme de Arginine, Glycine, Acide aspartique [-RGDfK-].

Ces observations ont ainsi servi de base au développement de molécules de ciblage spécifique de l'intégrine αᵥβ₃.

Une publication de Boturyn et al. "Template Assembled Cyclopeptides as Multimeric System for Integrin Targeting and Endocytosis", J. Am. Chem. Soc. 2004, 126, 5730-5739, présente en outre des traceurs fluorescents spécifiques de l'intégrine αᵥβ₃. Le traceur de Boturyn comprend un châssis RAFT configuré de sorte à définir un plan moyen, tel que défini dans le brevet de P. Dumy cité précédemment, permettant d'une part du plan moyen la fixation de molécules de ciblage, et, d'autre part du plan moyen la fixation du fluorophore directement sur un résidu Lysine de la face dite inférieure du châssis RAFT. En l'espèce, les molécules de ciblage comprennent quatre cyclopentapeptides comprenant la séquence peptidique RGD. Le fluorophore est la fluorescéine qui fluoresce dans le domaine de longueurs d'onde du visible.

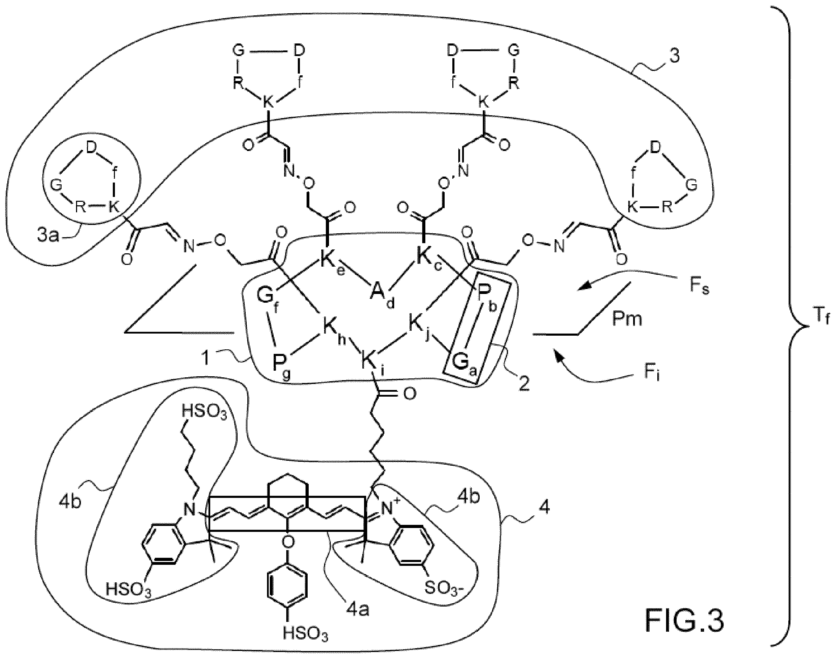

Elisabeth Garanger : "Conception, Synthèse et Caractérisation de Nouveaux Systèmes de Guidage et de Vectorisation pour la Cancérologie", Thèse - Université Joseph Fourier - Grenoble I, 26 juillet 2005, décrit le traceur (Cy5)RAFT(c[-RGDfK-])₄. Sur la base de ces développements, il a été proposé un traceur fluorescent dans le domaine du proche infrarouge spécifique de l'intégrine αᵥβ₃, représenté sur la figure 3.

Le traceur fluorescent **Tf** comprend :
- un châssis moléculaire **1,** en l'espèce, le décapeptide cyclique RAFT comprenant la séquence de dix résidus d'acides aminés : [-Gₐ-P_{b}-K_{c}-A_{d}-Kₑ-G_{f}-P_{g}-Kₕ-Kᵢ-Kⱼ] décrite à la figure 1,
- un fluorophore **4** de la famille des cyanines fluoresçant dans le domaine de longueurs d'onde compris entre 700 et 900 nm, et
- un ensemble de ciblage **3** de l'intégrine αᵥβ₃. En l'espèce, l'ensemble de ciblage **3** comprend quatre molécules de ciblage **3a** comprenant le cyclo-pentapeptide comprenant la séquence de résidus d'acides aminés [-RGD-].

Les molécules cyclo-pentapeptides **3** sont couplés aux quatre résidus d'acides aminés Lysine **K_{c}, Kₑ, Kₕ** et **Kⱼ** de la face dite supérieure **Fₛ** du châssis moléculaire **1** via des liaisons oximes.

Le fluorophore **4** comprend notamment une chaîne carbonée **4a** présentant des doubles liaisons covalentes et un groupement aromatique indol **4b** aux extrémités de la chaîne carbonée **4a** disposés de sorte à permettre une délocalisation des électrons des doubles liaisons sur l'ensemble de la chaîne carbonée **4a** et des groupements aromatiques indol **4b.**

Notons que le fluorophore **4** est ici l'IRDye800 (marque déposée) mais peut également être de la cyanine 5 (marque déposée), l'Alexa fluor 700 (marque déposée)ou un fluorophore développé spécifiquement à 700 nm noté BM 105 (marque déposée).

Le fluorophore **4** est relié à un résidu lysine **Kᵢ** de la face dite inférieure **Fᵢ** du châssis moléculaire **1** en formant une liaison amide via un groupement aliphatique d'un des groupements aromatiques indol **4b.**

Le procédé de réalisation du traceur fluorescent **Tf** selon l'état de la technique comprend :
- une première étape de synthèse du châssis RAFT **1** comprenant :
   ∘ une sous-étape de synthèse d'un décapeptide linéaire sur résine [-K(boc)-K(Alloc)-K(Boc)- P-G-K(Boc)- A-K(Boc)-P-G],
   ∘ une sous-étape de cyclisation du décapeptide en solution,
   ∘ une sous-étape de déprotection des K(Boc),
   ∘ une sous-étape de greffage d'un précurseur d'oxyamine protégé,
   ∘ une sous-étape de déprotection de K(Alloc).
- une deuxième étape de synthèse de la molécule de ciblage **3,** ici, le pentapeptide cyclique [-D(tBu)-f-K(Alloc)-R(Pbf)-G-] **3a** ,
- une troisième étape de couplage entre le châssis RAFT 1 et des molécules de ciblage **3a** comprenant :
   ∘ une sous-étape de cyclisation du pentapeptide en solution,
   ∘ une sous-étape de déprotection de K(Alloc),
   ∘ une sous-étape de greffage de S protégé,
   ∘ une sous-étape de déprotection de D, de S et de R, et
   ∘ une sous-étape d'oxydation de S,
- une quatrième étape d'activation du fluorophore **4.** En pratique, le fluorophore est commercialisé sous la forme activée ce qui le rend particulière instable et réactif vis-à-vis des amines.
- une cinquième étape de couplage, ultérieure aux troisième et quatrième étapes, entre le châssis RAFT comprenant la molécule de ciblage **3a** RAFT[-RGD-] et le fluorophore **4.**

Les rendements de ce procédé sont relativement faibles et notamment le rendement de couplage entre le châssis RAFT **1** comprenant les molécules de ciblage et le fluorophore **4.**

En effet, pour obtenir 15g de traceur fluorescent **Tf** tel que celui décrit figure 3, 410 g de molécule de ciblage **3a,** 75g de châssis RAFT **1** et 7g de fluorophore **4** sont nécessaires.

Les coûts globaux de réalisation d'un traceur fluorescent **Tf** selon l'art connu sont très élevés en raison d'une part du coût d'achat du fluorophore **4,** et, d'autre part du procédé de synthèse qui génère des pertes de matières premières importantes.

### RESUME DE L'INVENTION

Aussi, un but de l'invention est de proposer un traceur fluorescent ciblant ayant un coût très inférieur à celui d'un traceur fluorescent selon l'art connu

Selon un aspect de l'invention, il est proposé un procédé de synthèse d'un traceur fluorescent pour ciblage de tumeurs, ledit traceur fluorescent comprenant:
- au moins un premier fluorophore comprenant une chaîne carbonée (4a) comprenant au moins un enchaînement d'au moins trois doubles liaisons covalentes carbone-carbone de sorte à permettre une délocalisation des électrons des doubles liaisons lorsque le fluorophore est excité par un rayonnement lumineux de manière à fluorescer avec un pic d'émission compris entre 770 nm et 870 nm
- un ensemble de ciblage **(3)** d'intégrine comprenant au moins deux molécules de ciblage identiques **(3a)** comprenant des pentapeptides cycliques, la séquence peptidique des pentapeptides ciblant une intégrine comprenant les résidus d'acides aminés [Arginine - Glycine - Acide aspartique-] (RGD), et
- un decapeptide cyclique **(1)** :
   - configuré de sorte à définir un plan moyen **(Pm)** définissant une première face supérieure **(Fₛ)** et une deuxième face inférieure **(Fᵢ),**
   - comprenant au moins un premier résidu d'acide aminé Lysine **(Kᵢ)** sur la deuxième face inférieure **(Fᵢ),**
   - les molécules de ciblage **(3a)** étant fixées sur la première face supérieure **(Fₛ)** du plan moyen **(Pm),**
   - le fluorophore étant fixé sur la deuxième face inférieure **(Fᵢ)** du plan moyen **(Pm)** via un bras espaceur **(8)** reliant un carbone de l'enchaînement des au moins trois doubles liaisons covalentes carbone-carbone et le résidu d'acide aminé lysine **(Kᵢ)** du décapeptide **(1)** ;
   ledit procédé comprenant au moins les étapes suivantes :
   - une étape de préparation d'un fluorophore modifié **(4')** dans lequel un bras espaceur (8) est couplé à au moins un premier fluorophore **(4)** comprenant une chaîne carbonée **(4a)** comprenant au moins un enchaînement d'au moins trois doubles liaisons covalentes carbone-carbone de sorte à permettre une délocalisation des électrons des doubles liaisons lorsque le fluorophore **(4)** est excité par un rayonnement lumineux de manière à fluorescer dans un domaine de longueurs d'ondes compris entre 700 et 1000 nm ;
   - une étape dans laquelle ledit decapeptide cyclique **(1)** est couplé audit fluorophore modifié **(4')** ;
   - une étape de couplage entre un ensemble de ciblage **(3)** comprenant au moins deux molécules de ciblage identiques **(3a),** et ledit decapeptide cyclique **(1)** couplé audit fluorophore modifié **(4').**

Selon un autre aspect de l'invention, il est proposé un traceur fluorescent obtenu selon ce procédé.

Le traceur fluorescent selon l'invention permet notamment d'utiliser des fluorophores commerciaux moins onéreux que ceux utilisés dans le procédé de l'état de la technique.

Avantageusement, ledit bras espaceur est apte à augmenter la délocalisation des électrons de ladite chaîne carbonée.

Le traceur fluorescent est facilement éliminé ce qui limite sa toxicité pour l'organisme.

Avantageusement, la première face et la deuxième face du décapeptide sont fonctionnalisables de manière chimio-sélective.

Préférentiellement, le décapeptide comprend l'enchaînement de résidus d'acides aminés suivants : [-K-K-K-P-G-K-A-K-P-G-].

Avantageusement, le bras espaceur comprend une chaîne carbonée linéaire comprenant un nombre de carbones supérieur ou égal à quatre permettant ainsi de limiter le problème d'espacement ou d'encombrement stérique lors du couplage entre le fluorophore et le châssis.

Avantageusement, le bras espaceur est relié au fluorophore via une liaison choisie parmi une liaison amide, éther et thioéther.

Avantageusement, le bras espaceur est relié au decapeptide via une liaison amide.

Avantageusement, le fluorophore est de la famille des cyanines et préférentiellement, le fluorophore est choisi parmi les fluorophores suivants : S0121, S0306, S0456, S2180, IR775 chloride, IR780 iodide, IR786, IR806, IR820.

Avantageusement, le decapeptide comprend un deuxième résidu Lysine sur la face inférieure, un deuxième fluorophore est accroché audit résidu lysine via un bras espaceur ou non. Les deux fluorophores peuvent alors interagir de sorte à permettre une fluorescence FRET acronyme de « Fröster Resonance Energy Transfer », en langue anglaise ou transfert d'énergie entre molécules fluorescentes, en langue française.

Avantageusement, les molécules de ciblage comprennent un peptide ou pseudo-peptide. Les molécules de ciblage comprennent des pentapeptides cycliques, la séquence peptidique du pentapeptide ciblant une intégrine comprend les résidus d'acides aminés [-Arginine- Glycine - Acide aspartique-] (RGD).

Avantageusement, le decapeptide cyclique comprend des résidus Lysine sur la première face supérieure sur lesquels les molécules de ciblage sont fixées via une liaison oxime. Ce type de liaison confère au traceur fluorescent une bonne stabilité in vivo et in vitro.

Alternativement, les molécules de ciblage sont fixées via des liaisons amide.

Alternativement, le decapeptide comprend au moins un résidu Cystéine sur la première face supérieure sur lequel une molécule de ciblage est fixée via une liaison thioéther permettant une fixation régio-sélective de molécules de ciblage différentes.

### LISTE DES FIGURES

L'invention sera mieux comprise et d'autres avantages apparaîtront à la lecture de la description qui va suivre donnée à titre non limitatif, et, grâce aux figures annexées parmi lesquelles :
- la figure 1, déjà décrite, représente un châssis RAFT,
- la figure 2, déjà décrite, illustre une molécule de ciblage comprenant un pentapeptide [-RGDfK-] cyclique spécifique de l'intégrine αᵥβ₃.
- la figure 3, déjà décrite, représente un traceur fluorescent selon l'art connu,
- la figure 4 illustre un représentant de la famille de traceurs fluorescents selon l'invention,
- les figures 5a, 5b, 5c représentent les formules générales de fluorophores utilisés dans un traceur selon l'invention,
- les figures 6a, 6b, 6c, 6d, 6e, 6f, 6g, 6h représentent des exemples de formules chimiques de fluorophores commerciaux potentiellement utilisables selon l'invention,
- les figures 7a, 7b, 7c et 7d représentent des bras espaceurs utilisés dans un traceur selon l'invention,
- la figure 8 représente un châssis RAFT permettant le greffage de deux fluorophores sur la face inférieure **Fi.**
- la figure 9 représente les réactions de couplage entre un bras espaceur et un fluorophore selon l'invention,
- les figures 10a et 10b représentent les réactions de synthèse du châssis RAFT, et de couplage entre le fluorophore muni du bras espaceur et le châssis RAFT, selon l'invention,
- la figure 11 représente les réactions de synthèse de la molécule de ciblage, selon l'invention,
- la figure 12 représente la réaction de couplage entre le châssis RAFT fluorescent et les molécules de ciblage via une liaison amide, selon l'invention ;
- la figure 13a illustre la distribution tissulaire d'un traceur Tf selon l'art connu et la figure 13b illustre la distribution tissulaire d'un traceur Tf selon un mode de réalisation de l'invention.

### DESCRIPTION DETAILLEE

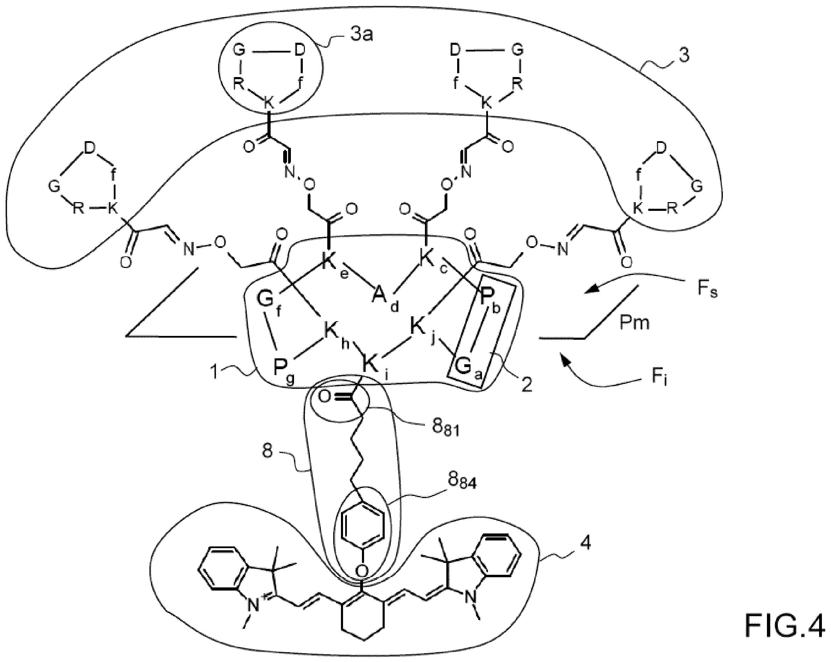

La **figure 4** est une formule semi-développée d'un représentant de la famille de traceur fluorescent Tf selon l'invention.

En l'espèce, le traceur fluorescent **Tf** comprend un châssis RAFT **1** comprenant, comme dans le document de Boturyn, un enchaînement de dix résidus d'acides aminés [-K-K-K-P-G-K-A-K-P-G-] sous forme cyclique. Les enchaînements des résidus d'acides aminés [-Glycine-Proline] constituent des coudes **2** de sorte à définir un plan moyen Pm de part et d'autre duquel un fluorophore **4** et un ensemble de ciblage **3** peuvent être fixés. De manière générale, les molécules de ciblage 3 peuvent être des molécules de ciblage des tumeurs. Ces molécules peuvent être des peptides, et plus particulièrement des peptides aptes à cibler au moins une intégrine, préférentiellement αᵥβ₃.

En l'occurrence, l'ensemble de ciblage **3** comprend des molécules de pentapeptide cyclique **3a** comprenant une séquence spécifique de type RGD. Les pentapeptides cycliques **3a** sont reliés au châssis RAFT **1** au niveau des résidus Lysine **K_{c}, Kₑ, Kₕ** et **Kⱼ** de la face supérieure **Fₛ** du châssis RAFT **1.** En l'espèce, ils sont reliés via une liaison oxime permettant une bonne stabilité in vivo et in vitro. Toutefois, il est aussi possible de les relier via une liaison amide. Alternativement, il est possible de relier au moins une molécule de ciblage 3a via une liaison thioéther à condition de remplacer un résidu Lysine par un résidu Cystéine auquel cas il est possible de greffer un type de molécules de ciblage sur les résidus Lysine et un autre type de molécule sur le résidu Cystéine permettant ainsi le greffage régio-sélectif de molécules de ciblage.

Le fluorophore **4** est fixé au châssis **1** via un bras espaceur **8.** En l'espèce, le fluorophore **4** est le IR775 (marque déposée) ou 2-[(E)-2-{(3E)-2-chloro-3-[(2Z)-2-(1,3,3-trimethyl-1,3-dihydro-2H-indol-2-ylidene)ethylidene]cyclohex-1-en-1-yl}ethenyl]-1,3,3-trimethyl-3H-indolium chloride, selon la nomenclature.

Le bras espaceur **8** comprend un enchaînement de cinq carbones, une fonction phénolate à une extrémité **8₈₄** de la chaîne carbonée reliant le bras espaceur **8** au fluorophore **4** et une fonction carboxylique **8₈₁** reliant le bras espaceur **8** au châssis RAFT **1.**

Plus précisément, les figures **5a, 5b** et **5c** représentent des formules générales de fluorophores **4** potentiellement utilisables selon l'invention.

Le fluorophore **4** selon l'invention doit posséder des propriétés d'absorption dans le domaine de longueurs d'ondes du proche infrarouge compris entre 750 nm et 1000 nm et un pic d'émission présentant un maximum compris entre 770 nm et 870 nm. Aussi, le fluorophore **4** présente un chaîne carbonée linéaire ou non comprenant un enchaînement de doubles liaisons covalentes conjuguées, les électrons des doubles liaisons pouvant se délocaliser. De plus, le fluorophore **4** présente sur l'un des carbones de la chaîne carbonée un groupement halogène **X** pouvant réagir avec un groupement.

La **figure 5a** présente un fluorophore **4** comprenant une chaîne linéaire de carbone **4a** présentant des doubles liaisons conjuguées, les électrons des doubles liaisons pouvant se délocaliser sur l'ensemble de la chaîne carbonée **4a.** Les extrémités de la chaîne carbonée **4a** présentent des groupements amine tertiaire et ammonium quaternaire.

La **figure 5b** présente aussi un fluorophore **4** potentiellement utilisable pour la réalisation d'un traceur fluorescent selon l'invention. La **figure 5b** diffère de la **figure 5a** en ce que l'extrémité ammonium quaternaire présente un groupement aromatique. La figure 5c présente à chacune des extrémités de la chaîne carbonée **4a** un groupement aromatique.

Eventuellement, le groupement aromatique tel que le pyrrole ou l'indol comprend un groupement aliphatique présentant un groupement fonctionnel pouvant modifier les caractéristiques physico-chimiques du traceur fluorescent **Tf.** Par exemple, l'ajout d'un groupement sulfonate peut augmenter l'hydrophilie et ainsi augmenter la solubilité du traceur fluorescent **Tf** dans un solvant aqueux.

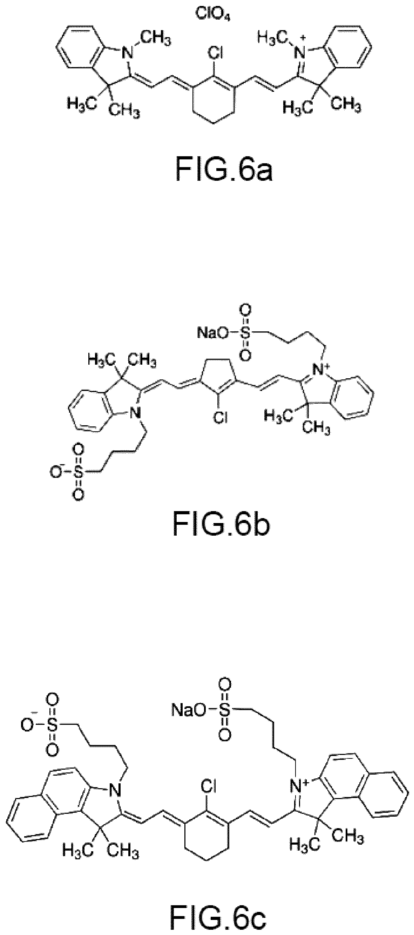

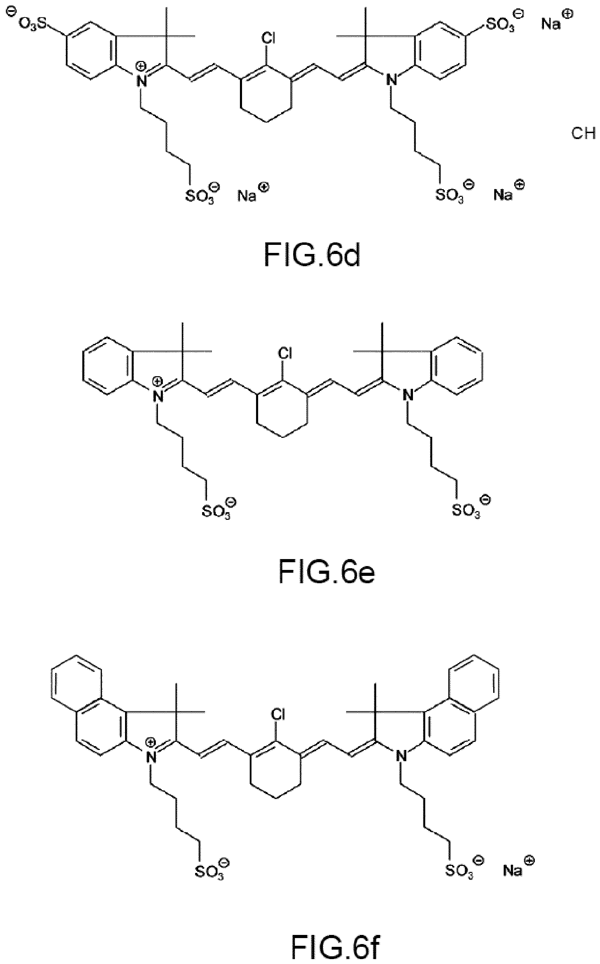

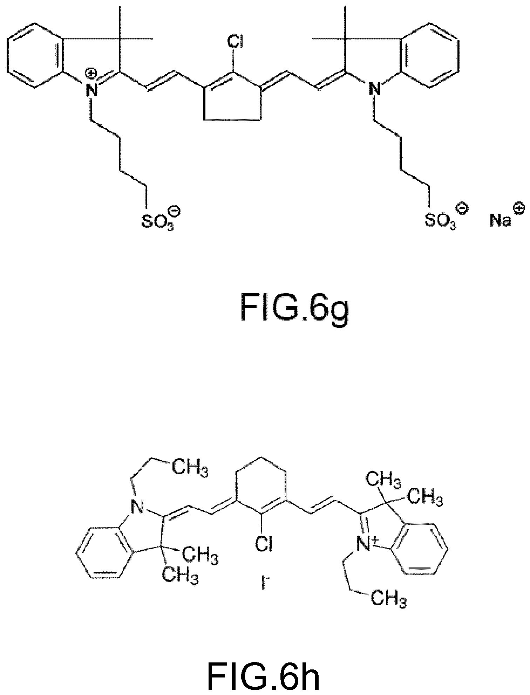

Quelques exemples de fluorophores du commerce potentiellement utilisables sont représentés sur les figures 6 :
- IR786 (marque déposée), ou perchlorate de 2-(2-[2-Chloro-3-([1,3-dihydro-1,3,3-trimethyl-2*H*-indol-2-ylidene]ethylidene)-1-cyclohexen-1-yl]ethenyl)-1,3,3-trimethylindolium (figure 6a),
- IR806 (marque déposée) ou sel interne (sel sodique) hydroxyde de 2-[2-[2-Chloro-3-[[1,3-dihydro-1,1-dimethyl-3-(4-sulfobutyl)-2*H*-benzo[e]indol-2-ylidene]-ethylidene]-1-cyclohexen-1-yl]-ethenyl]-1,1-dimethyl-3-(4-sulfobutyl)-1*H*-benzo[e]indolium(figure 6b),
- IR820 (marque déposée) ou sel interne (sel sodique) hydroxyde de 2-[2-[2-Chloro-3-[[1,3-dihydro-1,1-dimethyl-3-(4-sulfobutyl)-2*H*-benzo[e]indol-2-ylidene]-ethylidene]-1-cyclohexen-1-yl]-ethenyl]-1,1-dimethyl-3-(4-sulfobutyl)-1*H*-benzo[e]indolium (figure 6c),
- S0456 (marque déposée) ou sel interne (sel trisodique) hydroxyde de 3,3-Dimethyl-2-[2-[2-chloro-3-[2-[1,3-dihydro-3,3-dimethyl-5-sulfo-1-(4-sulfobutyl)-2H-indol-2-ylidene]-ethylidene]-1-cyclohexen-1-yl]-ethenyl]-5-sulfo-1-(4-sulfobutyl)-3H-indolium (figure 6d),
- S2180 (marque déposée) ou sel interne (sel trisodique) hydroxyde de 2-[2-(2-Chloro-3-[2-[1,1-dimethyl-7-sulfo-3-(4-sulfobutyl)-1,3-dihydro-benzo[e]indol-2-ylidene]-ethylidene]-cyclohex-1-enyl)-vinyl]-1,1-dimethyl-7-sulfo-3-(4-sulfobutyl)-1H-benzo[e]indolium (figure 6e),
- S0306 (marque déposée) ou sel interne (sel sodique) hydroxyde de 2-[2-[2-Chloro-3-[[1,3-dihydro-1,1-dimethyl-3-(4-sulfobutyl)-2H-benzo[e]indol-2-ylidene]-ethylidene]-1-cyclohexen-1-yl]-ethenyl]-1,1-dimethyl-3-(4-sulfobutyl)-1H-benzo[e]indolium (figure 6f),
- S0121 (marque déposée) ou sel interne (sel sodique) hydroxyde de 2-[2-[2-Chloro-3-[2-[1,3-dihydro-3,3-dimethyl-1-(4-sulfobutyl)-2H-indol-2-ylidene]-ethylidene]-1-cyclohexen-1-yl]-ethenyl]-3,3-dimethyl-1-(4-sulfobutyl)-3H-indolium (figure 6g),
- IR780 iodide (marque déposée) ou iodure de 2-[2-[2-Chloro-3-[(1,3-dihydro-3,3-dimethyl-1-propyl-2*H*-indol-2-ylidene)ethylidene]-1-cyclohexen-1-yl]ethenyl]-3,3-dimethyl-1-propylindolium (figure 6h).

Ces fluorophores ont un coût bien plus faible que les fluorophores utilisés pour la synthèse de traceurs fluorescents **Tf** selon l'art antérieur.

La différence de coût peut s'expliquer d'une part par le fait que ces fluorophores sont structurellement plus simples que les fluorophores utilisés auparavant de type IRDye®800 et d'autre part par le fait qu'il n'est pas nécessaire de les activer.

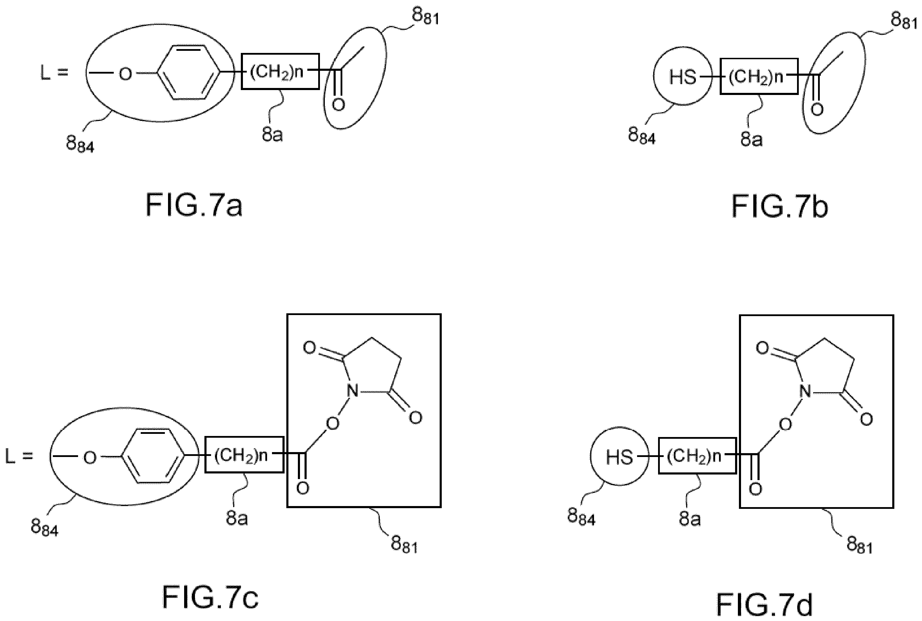

Par ailleurs, les figures **7a, 7b, 7c** et **7d** présentent des formules semi-développées de bras espaceur **8** potentiellement utilisables.

De manière générale, le bras espaceur **8** selon l'invention comprend une chaîne carbonée **8a,** un premier groupement terminal **8₈₄** reliant le fluorophore **4** au bras espaceur **8** et un deuxième groupement terminal **8₈₁** reliant le bras espaceur **8** au châssis RAFT **1.**

Bien que l'état de la technique présente un bras espaceur constitué d'une tyrosine, il a été démontré qu'il n'est pas possible de greffer un fluorophore **4** sur un châssis RAFT **1** selon l'invention via un bras espaceur constitué d'un résidu tyrosine à cause notamment de problèmes d'encombrement stérique ou d'espacement.

Préférentiellement, le nombre de carbones de la chaîne carbonée **8a** est supérieur ou égal à 4 facilitant ainsi le greffage du fluorophore **4** et limitant ainsi les problèmes liés à l'encombrement stérique.

Le premier groupement terminal **8₈₄** peut être un groupement amide (correspondant à un groupement amine avant une réaction entre le bras espaceur 8 et le fluorophore 4), un groupement éther (correspondant à un groupement alcool avant une réaction entre le bras espaceur 8 et le fluorophore 4), ou un groupement thioéther (correspondant à un groupement thiol avant une réaction entre le bras espaceur 8 et le fluorophore 4). La fixation du bras espaceur **8** via le premier groupement terminal **8₈₄** modifie sensiblement le maximum d'émission. En effet, un premier groupement terminal amine diminue la longueur d'onde du pic maximum de fluorescence de 100 à 150 nm. Aussi, ce type de premier groupement terminal ne sera pas privilégié. Un premier groupement terminal éther (figure 7a et figure 7c, correspondant à un groupement alcool avant une réaction entre le bras espaceur 8 et le fluorophore 4) diminue la longueur d'onde du maximum du pic d'émission de 10 à 15 nm. Un premier groupement terminal thioéther (figure 7b et figure 7d, correspondant à un groupement thiol avant une réaction entre le bras espaceur 8 et le fluorophore 4) augmente la longueur d'onde du maximum du pic d'émission de 10 à 15 nm. En outre, l'utilisation d'un premier groupement terminal de type phénol (figure 7a et figure 7c) augmente la délocalisation des électrons de la chaîne carbonée 4a du fluorophore ce qui augmente le rendement quantique. De manière générale, un groupement terminal 8₈₄ du bras espaceur est apte à augmenter la délocalisation des électrons de la chaîne carbonée 4a du fluorophore 4, et de cette manière à augmenter le rendement quantique.

Le deuxième groupement terminal **8₈₁** peut être un acide carboxylique (figure 7a et figure 7b) ou succinimide ester (figure 7c et figure 7d).

Le bras espaceur **8** est fixé via le deuxième groupement terminal **8₈₁** sur un résidu Lysine situé sur la face dite inférieure **F**ᵢ du châssis RAFT **1.**

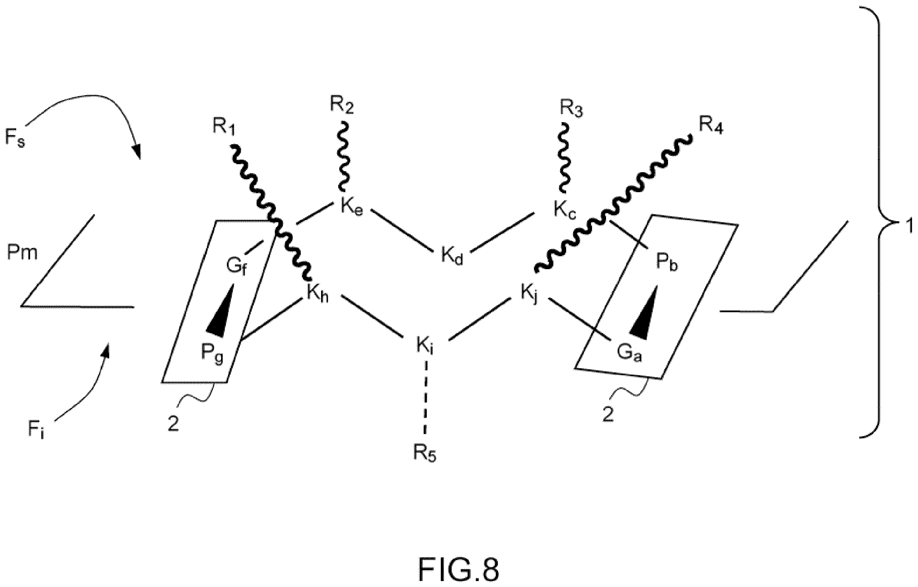

Selon une variante de l'invention représentée sur la figure 8, le résidu Alanine situé sur la face dite inférieure **Fᵢ** du châssis RAFT **1** peut être substitué par un résidu Lysine, auquel cas un deuxième fluorophore peut accessoirement être fixé de sorte à permettre une fluorescence FRET, par exemple.

Selon un autre aspect de l'invention, il est proposé un procédé de réalisation du traceur **Tf** comprenant une étape de couplage du fluorophore **4** avec le châssis RAFT **1** de sorte à former un châssis RAFT fluorescent préalablement à l'étape de couplage des molécules de ciblage **3.**

Ce procédé de réalisation diminue les pertes de matières premières, et plus particulièrement celles des molécules de ciblage, et permet d'éviter l'étape d'activation du fluorophore avant l'étape de couplage.

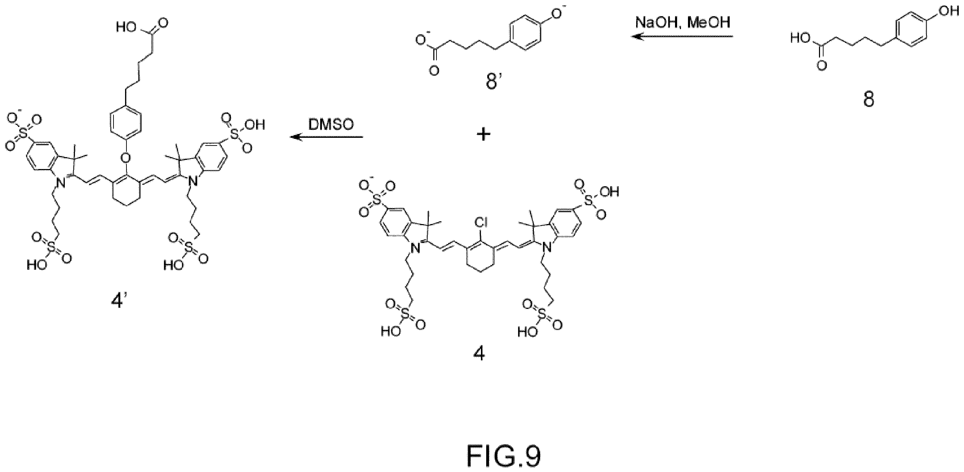

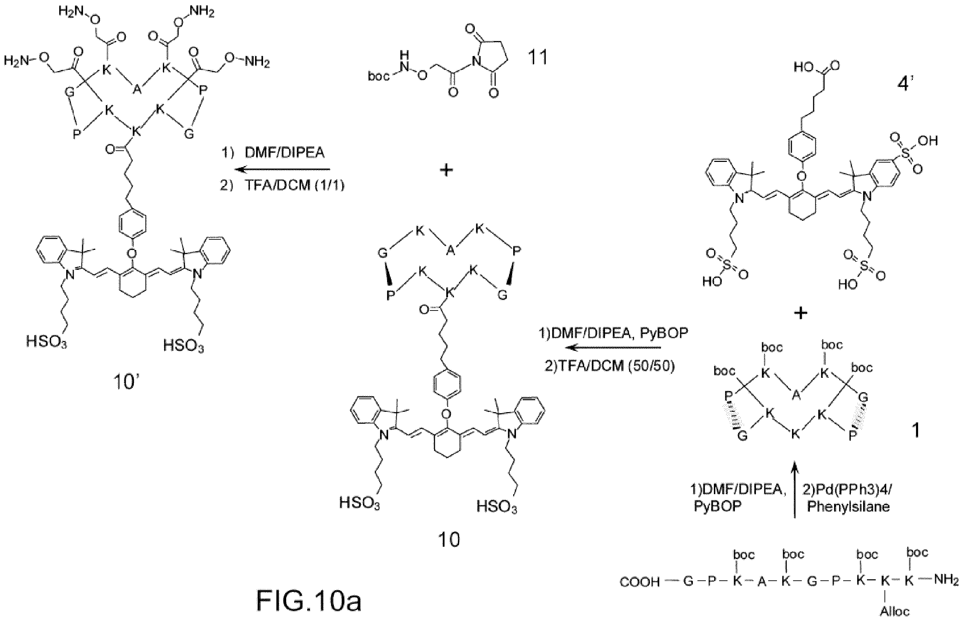

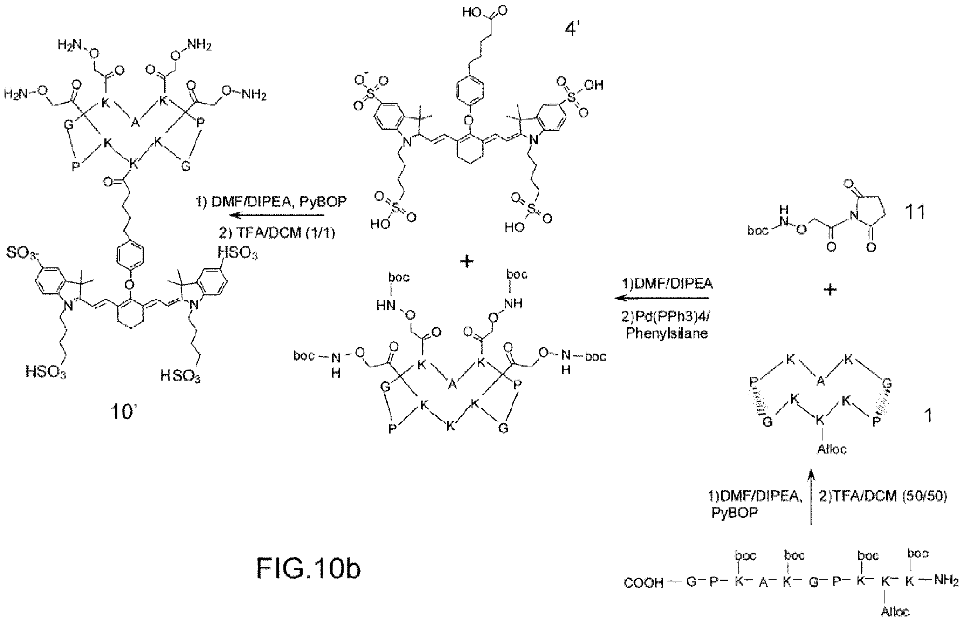

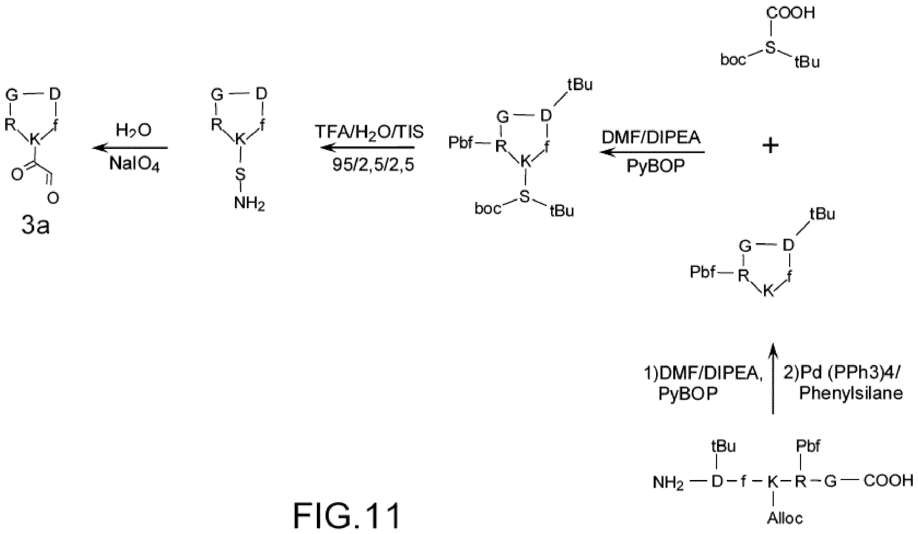

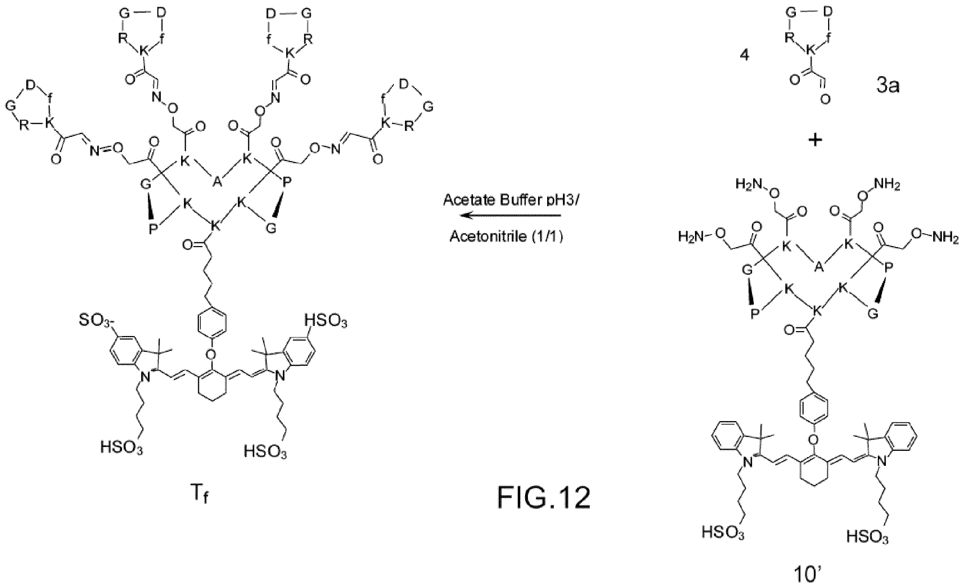

Le procédé comprend :
- une première étape, illustrée sur la figure 9, de préparation d'un fluorophore modifié **4'** dans laquelle le bras espaceur **8** est couplé au fluorophore **4,**
- une deuxième étape, représentée sur la figure 10, dans laquelle le châssis RAFT **1** est synthétisé,
- une troisième étape, représentée sur la figure 10, dans laquelle le châssis RAFT **1** est couplé au fluorophore modifié **4'** de sorte à former un châssis fluorescent **10,**
- une quatrième étape, illustrée sur la figure 10, dans laquelle un précurseur **11** de liaison oxime est greffé au châssis RAFT **1** sur les résidus Lysine de la face dite supérieure **Fₛ** de sorte à former un châssis fluorescent modifié **10',**
- une cinquième étape, représentée sur la figure 11, dans laquelle les molécules de ciblage **3a** sont synthétisées, et
- une sixième étape, illustrée sur la figure 12, de couplage entre les molécules de ciblage **3a** et le châssis fluorescent modifié **10'.**

Plus précisément, la figure 9 présente les étapes de préparation du fluorophore modifié **4',** décrite notamment dans le document de Hyun, H. et al, « c-GMP-compatible preparative scale of near-infrared fluorophores. Contrast Media Mol. Imaging, 2012, 7 : 516 ». Le bras espaceur **8,** acide 5-(4-hydroxyphenyl) pentanoïque, comprend un premier groupement terminal **8₈₁** phénol. Le phénol est convertit en phénolate, plus réactif que le phénol, dans une solution d'hydroxyde de sodium dans le méthanol pour former un bras espaceur modifié **8'.** Le bras espaceur **8'** obtenu est ensuite mélangé au fluorophore **4,** ici le S0456 (ou sel interne (sel trisodique) hydroxyde de 3,3-Dimethyl-2-[2-[2-chloro-3-[2-[1,3-dihydro-3,3-dimethyl-5-sulfo-1-(4-sulfobutyl)-2H-indol-2-ylidene]-ethylidene]-1-cyclohexen-1-yl]-ethenyl]-5-sulfo-1-(4-sulfobutyl)-3H-indolium) dans le DMSO (acronyme de DiMéthylSulfOxyde) pour obtenir le fluorophore modifié **4'** constitué du fluorophore 4 sur lequel est greffé le bras espaceur **8'.**

La figure 10a présente les deuxième, troisième et quatrième sous-étapes du procédé. Dans un premier temps, un décapeptide linaire comprenant la séquence de résidus d'acides aminés [-K(Boc)-K(Alloc)-K(Boc)-P-G-K(Boc)-A-K(Boc)-P-G-] est synthétisé sur résine, les groupements BOC et ALLOC étant des groupements protecteurs de sorte à permettre, par la suite, une fonctionnalisation régiosélective du châssis fluorescent. Après décrochage de la résine, le décapeptide est cyclisé et le résidu Lysine protégé par le groupement (Alloc) est déprotégé en présence de palladium (Pd⁰) et de phénylsilane de sorte à former un châssis RAFT **1** présentant un plan moyen **Pm.** Le fluorophore modifié **4'** est ensuite greffé sur le résidu Lysine déprotégé. Les groupements protecteurs BOC sur les résidus Lysine de la face dite supérieure **Fₛ** sont clivés en milieu acide puis un précurseur d'oxyamine protégé **11** est greffé sur les résidus Lysine via une liaison amide pour former le châssis RAFT fluorescent modifié 10'. Notons que la troisième étape et la quatrième étape peuvent être interverties, comme illustré sur la figure 10b. En l'espèce, dans un premier temps, les groupements protecteurs BOC sur les résidus Lysine de la face dite supérieure **Fₛ** sont clivés en milieu acide puis des précurseurs d'oxyamine protégés **11** sont greffés sur les résidus Lysine via une liaison amide. Le résidu Lysine situé sur la face inférieure **Fᵢ** protégé par le groupement (Alloc) est déprotégé de sorte à permettre le greffage du fluorophore modifié 4' pour former un châssis fluorescent modifié 10'.

Par ailleurs, il est intéressant de noter que la sous-étape d'activation du fluorophore **4** indispensable dans le procédé selon l'état de la technique n'est pas nécessaire dans le procédé selon l'invention. Dans l'invention, l'étape d'activation du fluorophore 4 réalisée dans l'art antérieur est contournée en partie par une étape de préparation d'un fluorophore modifié 4', dans laquelle un bras 8 réagit avec un groupement halogène **X** présent sur l'un des carbones de la chaîne carbonée. De cette manière, le bras espaceur 8 relie un carbone de l'enchaînement des au moins trois doubles liaisons du fluorophore 4. Cet agencement permet une réduction du coût de synthèse du traceur à des performances équivalentes et/ou meilleures.

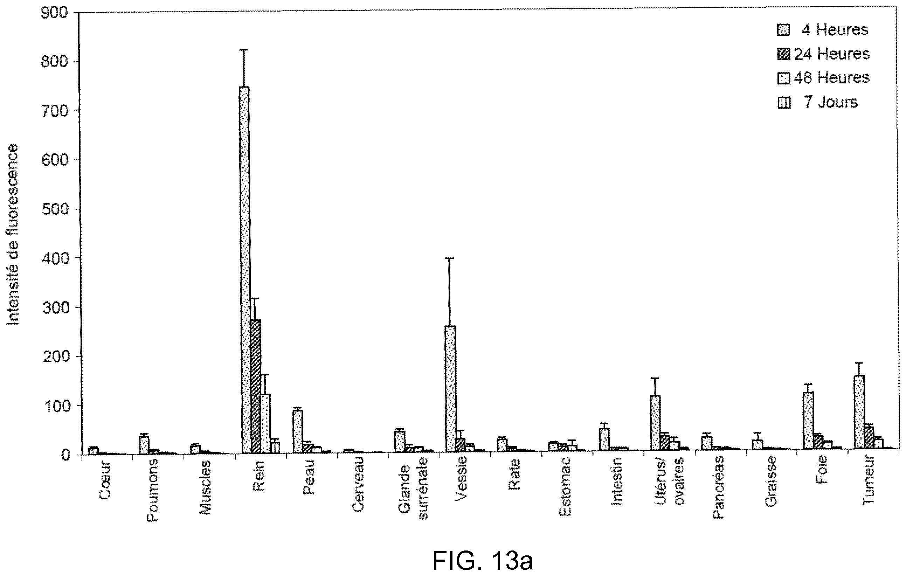

La figure 13a illustre la distribution tissulaire d'un traceur **Tf** selon l'art connu, décrit en figure 3, à différents temps post-injection.

En l'espèce, la fluorescence a été mesurée à des temps post-injection de 4h, 24h, 48h puis de sept jours, les organes ou tissus étudiés sont : le cœur, les poumons, les muscles, le rein, la peau, le cerveau, les glandes surrénales, la vessie, la rate, l'estomac, les intestins, les ovaires et l'utérus, le pancréas, les graisses, le foie et une tumeur mammaire murine sous-cutanée (Ts/Apc).

Le diagramme montre que l'intensité de fluorescence est la plus importante à un temps post-injection du traceur **Tf** selon l'art connu de 4h et diminue après 24h. Après sept jours, l'intensité de fluorescence dans les organes et tissus est quasi nulle.

En outre, le diagramme montre que le traceur **Tf** selon l'art connu est particulièrement bien approprié pour cibler une tumeur surexprimant l'intégrine αᵥβ₃. Ce diagramme montre également une accumulation importante du traceur **Tf** selon l'art connu dans les reins dès 4 heures post-injection, démontrant une élimination rapide du produit par voie rénale.

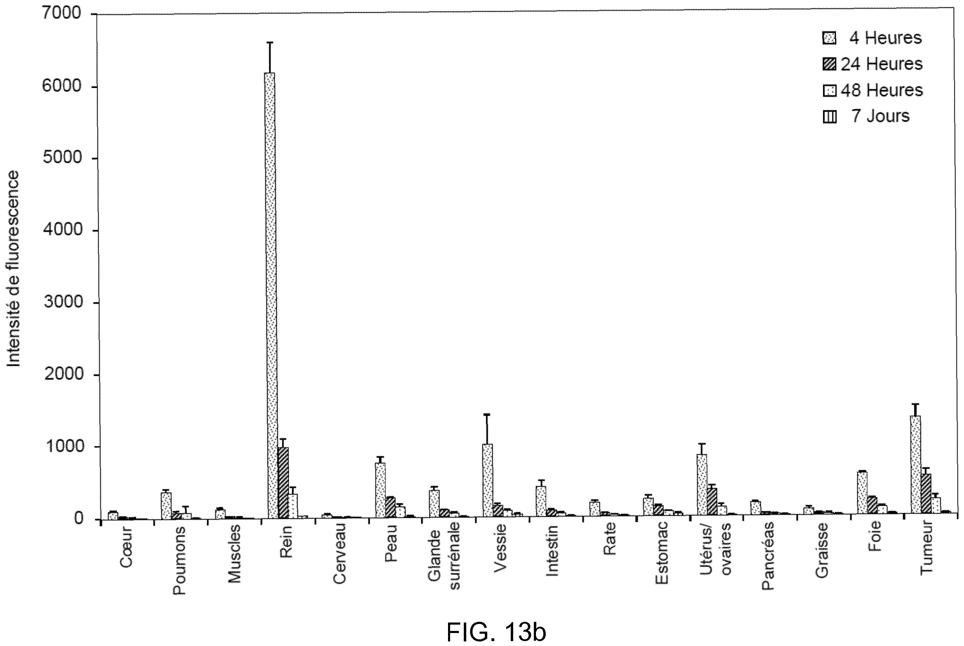

La figure 13b illustre la distribution tissulaire d'un traceur Tf selon un mode de réalisation de l'invention. La figure 13b illustre en particulier l'intensité de la fluorescence d'un traceur **Tf** selon un mode de réalisation de l'invention, par exemple illustré dans la figure 4, dans les mêmes organes et tissus que ceux étudiés figure 13a.

La distribution tissulaire du traceur **Tf** selon l'invention est similaire à la distribution observée pour le traceur **Tf** selon l'art connu.

Par ailleurs, l'affinité du traceur **Tf** selon l'invention est similaire à l'affinité d'un traceur **Tf** selon l'art connu tel que le Cy5-RAFT-(c[RGDfK])₄. Ceci s'explique par le fait que les molécules de ciblage sont identiques et représentées en quantités identiques dans le traceur **Tf** selon l'art connu et le traceur **Tf** selon l'invention.

Par contre, le traceur **Tf** selon l'invention présente une affinité bien plus élevée qu'un traceur monomérique présentant la molécule de ciblage représentée en un unique exemplaire.

Les figures 13a et 13b illustrent des performances similaires, équivalentes et/ou supérieures d'un traceur **Tf** selon un mode de réalisation de l'invention par rapport à un traceur **Tf** selon l'art connu.

La figure 11 présente les étapes de synthèse de la molécule de ciblage **3a.** Dans un premier temps, le pentapeptide linéaire comprenant la séquence de résidus d'acides aminés RGD spécifique des intégrines est synthétisé sur une résine. En l'espèce, le pentapeptide comprend la séquence [-D(tBu)-f-K(Alloc)-R(Pbf) G-]. Après décrochage du peptide de la résine, celui-ci est cyclisé puis le résidu Lysine protégé par le groupement Alloc est ensuite déprotégé en présence de palladium (Pd⁰) et de phénylsilane. Un résidu sérine protégée est ensuite greffé sur le résidu Lysine avant déprotection totale du pentapeptide en milieu acide. La fonction alcool de la sérine est ensuite oxydée avec du périodate de sodium dans l'eau pour obtenir un groupement aldéhyde.

La figure 12 illustre l'étape de couplage entre le châssis fluorescent modifié **10'** et les pentapeptides cycliques **3a.**

Dans le procédé revendiqué, le fluorophore commercial **4** intervient très tôt dans le processus de synthèse du traceur fluorescent **Tf** par rapport au procédé de l'état de la technique.

Or, les exigences de pureté et de qualité relatives aux matières premières incorporées dans des formulations destinées à l'administration humaine intervenant tôt dans le procédé de synthèse sont moindres que pour les matières premières intervenant en fin de procédé comme cela est le cas dans le procédé de l'état de la technique. En l'espèce, le fluorophore **4** peut donc être de qualité et de pureté inférieures à celles requises lors de la synthèse selon le procédé de l'état de la technique, ce qui contribue de manière significative à la baisse du coût d'achat du fluorophore **4.**

De plus, contrairement au procédé décrit dans l'état de la technique, l'étape d'activation du fluorophore préalable à l'étape de couplage entre le fluorophore modifié **10'** et le châssis RAFT **1** n'est pas nécessaire ce qui permet de diminuer encore les coûts de fabrication du traceur fluorescent **Tf** selon l'invention.

Aussi, la synthèse de 15 g de traceur fluorescent Tf requiert 30g de RAFT, 105g de RGD et 13g de fluorophore.

La nouvelle famille de traceurs fluorescents proposée dans la présente invention permet de diminuer de manière importante les coûts d'une part en permettant l'utilisation de matériaux moins onéreux mais aussi en améliorant le procédé de fabrication permettant ainsi de supprimer des étapes réactionnelles.

## Revendications

1. Procédé de synthèse d'un traceur fluorescent pour ciblage de tumeurs, ledit traceur fluorescent comprenant:
- au moins un premier fluorophore comprenant une chaîne carbonée **(4a)** comprenant au moins un enchaînement d'au moins trois doubles liaisons covalentes carbone-carbone de sorte à permettre une délocalisation des électrons des doubles liaisons lorsque le fluorophore est excité par un rayonnement lumineux de manière à fluorescer avec un pic d'émission compris entre 770 nm et 870 nm
- un ensemble de ciblage **(3)** d'intégrine comprenant au moins deux molécules de ciblage identiques **(3a)** comprenant des pentapeptides cycliques, la séquence peptidique des pentapeptides ciblant une intégrine comprenant les résidus d'acides aminés [-Arginine - Glycine - Acide aspartique-] (RGD), et
- un decapeptide cyclique **(1)** :
▪ configuré de sorte à définir un plan moyen **(Pm)** définissant une première face supérieure **(Fₛ)** et une deuxième face inférieure **(Fᵢ),**
▪ comprenant au moins un premier résidu d'acide aminé Lysine **(Kᵢ)** sur la deuxième face inférieure **(Fᵢ),**
▪ les molécules de ciblage **(3a)** étant fixées sur la première face supérieure **(Fₛ)** du plan moyen **(Pm),**
▪ le fluorophore étant fixé sur la deuxième face inférieure **(Fᵢ)** du plan moyen **(Pm)** via un bras espaceur **(8)** reliant un carbone de l'enchaînement des au moins trois doubles liaisons covalentes carbone-carbone et le résidu d'acide aminé lysine **(Kᵢ)** du décapeptide **(1)** ;
ledit procédé comprenant au moins les étapes suivantes :
- une étape de préparation d'un fluorophore modifié **(4')** dans lequel un bras espaceur **(8)** est couplé à au moins un premier fluorophore **(4)** comprenant une chaîne carbonée **(4a)** comprenant au moins un enchaînement d'au moins trois doubles liaisons covalentes carbone-carbone de sorte à permettre une délocalisation des électrons des doubles liaisons lorsque le fluorophore **(4)** est excité par un rayonnement lumineux de manière à fluorescer dans un domaine de longueurs d'ondes compris entre 700 et 1000 nm ;
- une étape dans laquelle ledit decapeptide cyclique **(1)** est couplé audit fluorophore modifié **(4')** ;
- une étape de couplage entre un ensemble de ciblage **(3)** comprenant au moins deux molécules de ciblage identiques **(3a),** et ledit decapeptide cyclique **(1)** couplé audit fluorophore modifié **(4').**

2. Procédé selon la revendication 1 dans lequel le fluorophore **(4)** est de la famille des cyanines.

3. Procédé selon la revendication 2 dans lequel le fluorophore **(4)** est choisi parmi les fluorophores suivants: S0121, S0306, S0456, S2180, IR775 chloride, IR780 iodide, IR786, IR806, IR820.

4. Procédé selon l'une des revendications 1 à 2, dans lequel ledit bras espaceur **(8)** est apte à augmenter la délocalisation des électrons d'une dite chaîne carbonée **(4a).**

5. Procédé selon la revendication 4 dans lequel la première face supérieure **(Fₛ)** et la deuxième face inférieure **(Fᵢ)** du décapeptide **(1)** sont fonctionnalisables de manière chimio-sélective.

6. Procédé selon la revendication 5 dans lequel le décapeptide comprend l'enchaînement de résidus d'acides aminés suivants : [-K-K-K-P-G-K-A-K-P-G-].

7. Procédé selon l'une des revendications précédentes dans lequel le bras espaceur **(8)** comprend une chaîne carbonée linéaire **(8a)** comprenant un nombre de carbone **(n)** supérieur ou égal à quatre.

8. Procédé selon l'une des revendications précédentes dans lequel le bras espaceur **(8)** est relié au fluorophore **(4)** via une liaison choisie parmi une liaison amide, éther, thioéther.

9. Procédé selon l'une des revendications précédentes dans lequel le bras espaceur **(8)** est relié au décapeptide cyclique **(1)** via une liaison amide.

10. Procédé selon l'une des revendications précédentes dans lequel l'oligopeptide comprend un deuxième résidu Lysine sur la face inférieure **(Fᵢ)** sur lequel un deuxième fluorophore **(4)** est accroché via un bras espaceur **(8)** ou non.

11. Procédé selon l'une des revendications précédentes dans lequel le décapeptide (1) comprend des résidus Lysine sur la première face supérieure **(Fₛ)** sur lesquels les molécules de ciblage (3a) sont fixées via une liaison oxime ou amide.

12. Procédé selon la revendication 11 dans lequel le décapeptide **(1)** comprend au moins un résidu Cystéine sur la première face supérieure **(Fₛ)** sur lequel est fixée une molécule de ciblage **(3a)** via une liaison thioéther.

13. Traceur fluorescent obtenu selon le procédé selon l'une des revendications 1 à 12.

## Patentansprüche

1. Verfahren zum Synthetisieren eines fluoreszierenden Tracers zum Targetieren von Tumoren, wobei der fluoreszierende Tracer Folgendes umfasst:
- mindestens einen ersten Fluorophor, der eine Kohlenstoffkette (4a) umfasst, die mindestens eine Kette von mindestens drei kovalenten Kohlenstoff-Kohlenstoff-Doppelbindungen umfasst, so dass eine Delokalisierung der Elektronen der Doppelbindungen möglich ist, wenn der Fluorophor durch eine Lichtstrahlung angeregt wird, so dass er mit einem Emissionspeak zwischen 770 nm und 870 nm fluoresziert,
- eine Integrin-Targeting-Anordnung (3), die mindestens zwei identische Targeting-Moleküle (3a) umfasst, die zyklische Pentapeptide umfassen, wobei die Peptidsequenz der ein Integrin targetierenden Pentapeptide die Aminosäurereste [-Arginin - Glycin - Asparaginsäure-] (RGD) umfasst, und
- ein zyklisches Dekapeptid (1):
▪ konfiguriert zum Definieren einer mittleren Ebene (Pm), die eine erste Oberseite (Fₛ) und eine zweite Unterseite (Fᵢ) definiert,
▪ mit mindestens einem ersten Lysin-Aminosäurerest (Kᵢ) auf der zweiten Unterseite (Fᵢ),
▪ wobei die Targeting-Moleküle (3a) auf der ersten Oberseite (Fs) der mittleren Ebene (Pm) befestigt sind,
▪ wobei der Fluorophor an der zweiten Unterseite (Fᵢ) der Mittelebene (Pm) über einen Spacerarm (8) fixiert ist, der einen Kohlenstoff der Kette der mindestens drei kovalenten Kohlenstoff-Kohlenstoff-Doppelbindungen und den Lysin-Aminosäurerest (Kᵢ) des Dekapeptids (1) verbindet;
wobei das Verfahren mindestens die folgenden Schritte umfasst:
- einen Schritt des Herstellens eines modifizierten Fluorophors (4'), bei dem ein Spacerarm (8) mit mindestens einem ersten Fluorophor (4) gekoppelt ist, der eine Kohlenstoffkette (4a) umfasst, die mindestens eine Kette von mindestens drei kovalenten Kohlenstoff-Kohlenstoff-Doppelbindungen umfasst, um eine Delokalisierung der Elektronen der Doppelbindungen zu ermöglichen, wenn der Fluorophor (4) durch eine Lichtstrahlung angeregt wird, so dass er in einem Wellenlängenbereich zwischen 700 und 1000 nm fluoresziert;
- einen Schritt, bei dem das zyklische Dekapeptid (1) an das modifizierte Fluorophor (4') gekoppelt wird;
- einen Schritt des Koppelns zwischen einer Targeting-Anordnung (3), die mindestens zwei identische Targeting-Moleküle (3a) umfasst, und dem zyklischen Dekapeptid (1), das mit dem modifizierten Fluorophor (4') gekoppelt ist.

2. Verfahren nach Anspruch 1, wobei der Fluorophor (4) zur Familie der Cyanine gehört.

3. Verfahren nach Anspruch 2, wobei der Fluorophor (4) aus den folgenden Fluorophoren ausgewählt ist: S0121, S0306, S0456, S2180, IR775 Chlorid, IR780 Jodid, IR786, IR806, IR820.

4. Verfahren nach einem der Ansprüche 1 bis 2, wobei der Spacerarm (8) die Elektronen-Delokalisation einer genannten Kohlenstoffkette (4a) erhöhen kann.

5. Verfahren nach Anspruch 4, wobei die erste Oberseite (Fₛ) und die zweite Unterseite (Fᵢ) des Dekapeptids (1) chemoselektiv funktionalisierbar sind.

6. Verfahren nach Anspruch 5, wobei das Dekapeptid die Kette der folgenden Aminosäurereste umfasst: [-K-K-K-P-G-K-A-K-P-G-].

7. Verfahren nach einem der vorherigen Ansprüche, wobei der Spacerarm (8) eine lineare Kohlenstoffkette (8a) mit einer Kohlenstoffzahl (n) von gleich oder größer als vier umfasst.

8. Verfahren nach einem der vorherigen Ansprüche, wobei der Spacerarm (8) mit dem Fluorophor (4) über eine Bindung verbunden ist, die aus einer Amid-, Ether-, Thioetherbindung ausgewählt ist.

9. Verfahren nach einem der vorherigen Ansprüche, bei dem der Spacerarm (8) über eine Amidbindung mit dem cyclischen Dekapeptid (1) verbunden ist.

10. Verfahren nach einem der vorherigen Ansprüche, bei dem das Oligopeptid auf der Unterseite (Fᵢ) einen zweiten Lysinrest aufweist, an den ein zweiter Fluorophor (4) über einen Spacerarm (8) gebunden ist oder nicht.

11. Verfahren nach einem der vorherigen Ansprüche, bei dem das Dekapeptid (1) auf der ersten Oberseite (Fₛ) Lysinreste aufweist, an denen die Targeting-Moleküle (3a) über eine Oxim- oder Amidbindung fixiert sind.

12. Verfahren nach Anspruch 11, wobei das Dekapeptid (1) mindestens einen Cysteinrest auf der ersten Oberseite (Fₛ) umfasst, an den ein Targeting-Molekül (3a) über eine Thioetherbindung gebunden ist.

13. Fluoreszierender Tracer, der mit dem Verfahren nach einem der Ansprüche 1 bis 12 erhalten wird.

## Claims

1. Method for synthetizing a fluorescent tracer for targeting tumors, said fluorescent tracer comprising:
- at least a first fluorophore comprising a carbon-based chain (4a) comprising at least a sequence of at least three covalent carbon-carbon double bonds, so as to enable delocalization of the electrons of the double bonds when the fluorophore is excited by light radiation so as to fluoresce with an emission peak of between 770 nm and 870 nm,
- an assembly (3) for targeting integrin comprising at least two identical targeting molecules (3a), comprising cyclic pentapeptides, the peptide sequence of the pentapeptides targeting an integrin comprise the amino acid residues [-arginine - glycine - aspartic acid-] (RGD), and
- a cyclic decapeptide (1):
▪ configured so as to define a mean plane (Pm) defining a first upper face (Fₛ) and a second lower face (Fᵢ),
▪ comprising at least a first lysine (Kᵢ) amino acid residue on the second lower face (Fᵢ),
▪ the targeting molecules (3a) being fixed to the first upper face (Fₛ) of the mean plane (Pm),
▪ the fluorophore being fixed to the second lower face (Fᵢ) of the mean plane (Pm) via a spacer arm (8) connecting a carbon of the sequence of the at least three covalent carbon-carbon double bonds and the lysine (Kᵢ) amino acid residue of the decapeptide (1);
said method comprising at least the following steps:
- a step of preparing a modified fluorophore (4'), wherein a spacer arm (8) is coupled to at least a first fluorophore (4) comprising a carbon-based chain (4a) comprising at least a sequence of at least three covalent carbon-carbon double bonds, so as to enable delocalization of the electrons of the double bonds when the fluorophore (4) is excited by light radiation, so as to fluoresce in a range of wavelengths of between 700 nm and 1000 nm;
- a step in which said cyclic decapeptide (1) is coupled to said modified fluorophore (4');
- a step of coupling a targeting assembly (3), comprising at least two identical targeting molecules (3a), and said cyclic decapeptide (1), which is coupled to said modified fluorophore (4').

2. Method according to claim 1, wherein the fluorophore (4) is from the cyanine family.

3. Method according to claim 2, wherein the fluorophore (4) is chosen from the following fluorophores: S0121, S0306, S0456, S2180, IR775 chloride, IR780 iodide, IR786, IR806, IR820.

4. Method according to one of claims 1 to 2, wherein said spacer arm (8) is able to increase delocalization of the electrons of a said carbon-based chain (4a).

5. Method according to claim 4, wherein the first upper face (Fₛ) and the second lower face (Fᵢ) of the decapeptide (1) are chemoselectively functionalizable.

6. Method according to claim 5, wherein the decapeptide comprises the sequence of the following amino acid residues: [-K-K-K-P-G-K-A-K-P-G-].

7. Method according to one of the preceding claims, wherein the spacer arm (8) comprises a linear carbon-based chain (8a) comprising a carbon number (n) greater than or equal to four.

8. Method according to one of the preceding claims, wherein the spacer arm (8) is connected to the fluorophore (4) via a bond chosen from an amide, ether or thioether bond.

9. Method according to one of the preceding claims, wherein the spacer arm (8) is connected to the cyclic decapeptide (1) via an amide bond.

10. Method according to one of the preceding claims, wherein the oligopeptide comprises a second lysine residue on the lower face (Fᵢ) to which a second fluorophore (4) is attached via a spacer arm (8) or not.

11. Method according to one of the preceding claims, wherein the decapeptide (1) comprises lysine residues on the first upper face (Fₛ), to which the targeting molecules (3a) are fixed via an oxime or amide bond.

12. Method according to claim 11, wherein the decapeptide (1) comprises at least one cysteine residue on the first upper face (Fₛ), to which a targeting molecule (3a) is fixed via a thioether bond.

13. Fluorescent tracer obtained by the method according to any one of claims 1 to 12.
